(19) Europäisches Patentamt
European Patent Office
Office européen des brevets

(11) **EP 1 731 147 A1**

(12) **EUROPEAN PATENT APPLICATION**
published in accordance with Art. 158(3) EPC


(43) Date of publication:
**13.12.2006 Bulletin 2006/50**

(21) Application number: **05727909.3**

(22) Date of filing: **30.03.2005**

(51) Int Cl.:
***A61K 31/22*** *(2006.01)* ***A61K 31/40*** *(2006.01)*
***A61K 31/47*** *(2006.01)* ***A61K 47/08*** *(2006.01)*
***A61K 47/10*** *(2006.01)* ***A61P 17/00*** *(2006.01)*

(86) International application number:
**PCT/JP2005/006038**

(87) International publication number:
**WO 2005/094814 (13.10.2005 Gazette 2005/41)**

(84) Designated Contracting States:
**AT BE BG CH CY CZ DE DK EE ES FI FR GB GR HU IE IS IT LI LT LU MC NL PL PT RO SE SI SK TR**

(30) Priority: **31.03.2004 US 557687 P**



(71) Applicants:
- **Kowa Co., Ltd.**
  **Naka-ku,**
  **Nagoya-shi,**
  **Aichi 460-8625 (JP)**
- **Nissan Chemical Industries, Ltd.**
  **Chiyoda-ku,**
  **Tokyo 101-0054 (JP)**

(72) Inventors:
- **NAKAI, Tatsuya**
  **Shizuoka 4170845 (JP)**
- **KANEBAKO, Makoto**
  **Sunto-gun,**
  **Shizuoka 411-0943 (JP)**

(74) Representative: **Hartz, Nikolai et al**
**Wächtershauser & Hartz**
**Weinstrasse 8**
**80333 München (DE)**


(54) **EXTERNAL PREPARATION**


(57) An external preparation comprising following ingredients (A) and (B) :
(A) a compound represented by the following general formula (1)

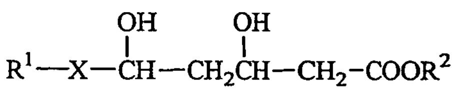

(1)

wherein $R^1$ represents an organic residue having a cyclic structure that may have a substituent; $R^2$ represents a hydrogen atom or a lower alkyl group; and X represents an ethylene group or an ethenylene group
or a salt thereof, and
(B) a monoterpene.

The external preparation of the present invention is excellent in percutaneous absorbability.


EP 1 731 147 A1

## Description

### Technical Field

The present invention relates to an external preparation comprising a statin or a salt thereof as an active ingredient, which is excellent in percutaneous absorbability.

### Background Art

It is known that statins such as pitavastatin and pravastatin have an excellent HMG-CoA reductase inhibitory activity and are useful as therapeutic agents for hyperlipidemia (Patent Documents 1 and 2) and for Alzheimer' s disease (Patent Documents 3 to 5). Statins have been already used or are under development as a pharmaceutical preparation for oral administration such as a tablet. Moreover, in recent years, it has been reported that statins are also effective in treating osteoporosis (Patent Document 6) or the like.

On the other hand, these HMG-CoA reductase inhibitors are known to be useful as having a topical effect on acne, psoriasis, dandruff (Patent Documents 7 and 8), body hair growth inhibition (Patent Document 9), and skin aging inhibition (Patent Documents 10 and 11). The development as an external preparation has been required.

However, when developing as an external preparation, it is difficult to make it transdermally absorb a drug because the skin has a barrier function of inhibiting perspiration from within or preventing any foreign matter from getting inside. Therefore, it has been required to improve percutaneous absorbability of an external preparation itself.

[Patent Document 1] Specification of U. S. Patent No. 5, 856, 336
[Patent Document 2] Specification of U.S. Patent No. 4, 346, 227
[Patent Document 3] Specification of U. S. Patent No. 6, 080, 778
[Patent Document 4] Specification of U.S. Patent No. 6, 472, 421
[Patent Document 5] Specification of U.S. Patent No. 6, 511, 800
[Patent Document 6] Specification of U.S. Patent No. 6, 022, 887
[Patent Document 71 Specification of U.S. Patent No. 5, 730, 992
[Patent Document 8] Specification of U.S. Patent No. 6, 126, 947
[Patent Document 9] SpecificationofU.S. Patent No. 5, 840, 752
[Patent Document 10] SpecificationofU.S. Patent No. 5, 733, 558
[Patent Document 11] Specification of U.S. PatentNo. 5, 902, 805

### Disclosure of the Invention

The object of the present invention is to provide an external preparation comprising a statin or a salt thereof as an active ingredient, which is excellent in percutaneous absorbability.

We were dedicated to studying for obtaining an external preparation comprising a statin or a salt thereof excellent in percutaneous absorbability. Consequently, we have found that an external preparation having an excellent percutaneous absorbability can be obtained by formulating a compound represented by the following general formula (1) known as an HMG-CoA reductase inhibitor in combination with a monoterpene to complete the present invention.

That is, the present invention is to provide an external preparation comprising following ingredients (A) and (B):

(A) a compound represented by the following general formula (1)

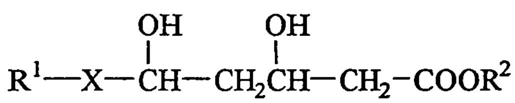

(1)

wherein $R^1$ represents an organic residue having a cyclic structure that may have a substituent; $R^2$ represents a hydrogen atom or a lower alkyl group; and X represents an ethylene group or an ethenylene group
or a salt thereof, and
(B) a monoterpene.
An external preparation of the present invention comprising a compound represented by general formula (1) or a salt thereof and a monoterpene is excellent in percutaneous absorbability.

A compound represented by general formula (1) or a salt thereof used in the present invention is a compound known as an HMG-CoA reductase inhibitor useful as a therapeutic agent for hyperlipidemia.

In general formula (1), examples of an organic residue having a cyclic structure represented by $R^1$ include indolyl, indenyl, pyridyl, pyrrolopyridyl, pyrazolopyridyl, thienopyridyl, pyrimidyl, pyrazolyl, pyrrolyl, imidazolyl, indolyzinyl, quinolyl, naphthyl, hexahydronaphthyl, cyclohexyl, phenylsilylphenyl, phenylthienyl, and phenylfuryl. Particularly preferred are hexahydronaphthyl, indolyl, pyridyl, pyrimidyl, pyrrolyl, or quinolyl.

Examples of substituents that can be contained in the above organic residues include hydroxy, alkyl, alkyloxyalkyl, alkylcarbonyloxy, alkylamino, alkylsulfonylamino, phenylsulfonylamino, carbamoyl that may be substituted by alkyl or phenyl, unsubstituted phenyl, halogenophenyl, alkylphenyl, alkoxyphenyl, and oxo. Among them, preferred are alkyl, alkyloxyalkyl, alkylcarbonyloxy, alkylamino, alkylsulfonylamino, phenylsulfonylamino, alkylcarbamoyl, phenylcarbamoyl, unsubstituted phenyl, halogenophenyl, alkylphenyl, and alkoxyphenyl.

Alkyl may be straight, branched, or cyclic, and has preferably 1 to 6 carbon atoms, and particularly preferably 1 to 4 carbon atoms. The same also applies to alkyl moieties of alkyloxyalkyl, alkylcarbonyloxy, alkylamino, alkylsulfonylamino, alkylphenyl, and alkoxyphenyl. Examples of alkyl include methyl, ethyl, propyl, isopropyl, butyl, isobutyl, sec-butyl, tert-butyl, cyclopropyl, cyclobutyl, cyclopentyl, and cyclohexyl.

Preferred alkyloxyalkyl has 2 to 7 carbon atoms. Examples thereof include methoxymethyl, methoxyethyl, ethoxymethyl, and ethoxyethyl.

Examples of alkylcarbonyloxy include acetyloxy, isobutylcarbonyloxy, and pivaloyloxy. Examples of alkylamino include methylamino and ethylamino. Examples of alkylsulfonylamino include methylsulfonylamino, ethylsulfonylamino, and N-methyl-N-methylsulfonylamino. Examples of alkylcarbamoyl include methylcarbamoyl and ethylcarbamoyl.

Examples of halogenophenyl include chlorophenyl, fluorophenyl, bromophenyl, and iodophenyl. Examples of alkylphenyl include methylphenyl and ethylphenyl. Examples of alkoxyphenyl include methoxyphenyl and ethoxyphenyl. A substitution position of a halogen atom, an alkyl group, or an alkoxy group on a phenyl group is not particularly limited, but preferably it is the para position.

Among the above $R^1$, isopropyl, cyclopropyl, or p-fluorophenyl is particularly preferable.

A lower alkyl group represented by $R^2$ in general formula (1) means an alkyl group having 1 to 6 carbon atoms. Examples thereof are those as described above.

A salt of a compound represented by general formula (1) is not particularly limited as long as it is a physiologically acceptable salt. Its specific examples include alkali metal salts such as sodium salts and potassium salts; alkali earth metal salts such as calcium salts and magnesium salts; organic amine salts such as phenethylamine salts; and ammonium salts. Among them, sodium salts and calcium salts are preferable, and calcium salts are particularly preferable.

Examples of the compound represented by general formula (1) include those described in the specification each of U.S. Patent No. 4, 739, 073, European Patent Application Laid-Open Nos. 114,027 and 367,895, U.S. Patent Nos. 5,001,255, 4,613,610, 4,851,427, 4,755,606, 4,808,607, 4,751,235, 4,939,159, 4,822,799, 4,804,679, 4,876,280, 4,829,081, 4, 927,-851, and 4,-588, 715; F. G. Kathawala, Medical Research Reviews, 11, 121-146 (1991); and the specification each of European Patent Application Laid-Open Nos. 304,063 and 330,057, U.S. Patent Nos. 5,026,708 and 4,868,185, European Patent Application Laid-Open Nos. 324,347 and 300,278, U.S. Patent Nos. 5, 013, 749, 5, 872, 130, 5,856,336, 4,231,938, 4,444,784, 4,346,227, 5,354,772, 5,273,995, 5,177,080, and 3, 983, 140, Japanese Patent No. 2, 648, 897, and U.S. Patent No. 5, 260, 440; Bioorganic & Medicinal Chemistry, 5, 437 (1977); and the specification each of Japanese Patent No. 2, 569, 746, European Patent No. 304, 063, and U.S. Patent No. 5, 856, 336.

Described in detail, preferred examples include lovastatin((+)-(1S,3R,7S,8S,8aR)-1,2,3,7,8,8a-hexahydro-3 , 7-dimethyl-8-[2-[(2R,4R)-tetrahydro-4-hydroxy-6-oxo-2H-p yran-2-yl]ethyl]-1-naphthyl (S)-2-methylbutyrate: specification of U.S. Patent No. 4,231,938); simvastatin ((+)-(1S,3R,7S,8S,8aR)-1,2,3,7,8,8a-hexahydro-3,7-dimethy 1-8-[2-[(2R, 4R)-tetrahydro-4-hydroxy-6-oxo-2H-pyran-2-yl] ethyl]-1-naphthyl 2,2-dimethylbutanoate: specification of U.S. Patent No. 4,444,784); pravastatin ((+)-(3R,5R)-3,5-dihydroxy-7-[(1S,2S,6S,8S,8aR)-6-hydroxy -2-methyl-8-[(S)-2-methylbutyryloxy]-1,2,6,7,8,8a-hexahyd ro-1-naphtyl]heptanoic acid: specification of U.S. Patent No. 4,346,227) ; fluvastatin ((3RS, 5SR,6E)-7-[3-(4-fluorophenyl)-1-(1-methylethyl)-1H-indol-2-yl]-3,5-dihydroxy-6-heptenoic acid: specification of U.S. Patent No. 5,354,772); atorvastatin ((3R,5R)-7-[2-(4-fluorophenyl)-5-isopropyl-3-phenyl-4-phe nylcarbamoyl-1H-pyrrol-1-yl]-3,5-dihydroxyheptanoic acid: specification of U.S. Patent No. 5,273,995); serivastatin ((3R,5S)-erythro-(E)-7-[4-(4-fluorophenyl)-2,6-diisopropy 1-5-methoxymethyl-pyridin-3-yl]-3,5-dihydroxy-6-heptenoic acid: specification of U. S. Patent No. 5, 177, 080) ; mevastatin ((+)-(1S,3R,7S,8S,8aR)-1,2,3,7,8,8a-hexahydro-7-methyl-8-[2-[(2R,4R)-tetrahydro-4-hydroxy-6-oxo-2H-pyran-2-yl]ethy 1]-1-naphthyl (S)-2-methylbutyrate: specification of U.S. Patent No. 3,983,140); rosuvastatin (7-[4-(4-fluorophenyl)-6-isopropyl-2-(N-methyl-N-methanes ulfonylaminopyrimidine)-5-yl]-(3R,5S)-dihydroxy-(E)-6-hep tenoic acid: specification each of U.S. Patent No. 5, 260, 440 and Japanese Patent No. 2,648,897); pitavastatin ((3R,5S,6E)-7-[2-cyclopropyl-4-(4-fluorophenyl)-3-quinoly 1]-3,5-dihydroxy-6-heptenoic acid: specification each of U.S. Patent No. 5, 856, 336 and Japanese Patent No. 2, 569, 746) ; or their salts. Specifically preferred examples include a salt of pitavastatin, a salt of atorvastatin, or a salt of pravastatin. Particularly preferred is pitavastatin calcium, atorvastatin calcium, or pravastatin sodium.

The content of a compound represented by general formula (1) or a salt thereof in the total amount of an external preparation is preferably 0. 001 to 20% by mass, more preferably 0.01 to 10% by mass, and particularly preferably 0.1 to 5% by mass.

A monoterpene used in the present invention stands for a generic designation of a compound that is formed by binding two isoprene units ($CH_2$=C ($CH_3$)-CH=$CH_2$) linearly or cyclically. Although monoterpenes include those having or not having an oxygen atom, a monoterpene having an oxygen atom is preferable. Examples of the monoterpene not having an oxygen atom include a hydrocarbon monoterpene. Examples of the monoterpene having an oxygen atom include a monoterpene having a hydroxyl group, a monoterpene having an aldehyde group, a monoterpene having a ketone group, a monoterpene having an oxide group, and a monoterpene having a carboxyl group. Preferred is a monoterpene having a hydroxyl group or a monoterpene having an aldehyde group.

Further, monoterpenes can be structurally classified intoalinearmonoterpene, amonocyclicmonoterpene, abicyclic monoterpene, a modified monoterpene, and the like. However, a linear monoterpene or a monocyclic monoterpene is preferable.

Specific examples of the linear monoterpene, monocyclic monoterpene, bicyclic monoterpene, and modified monoterpene include a linear monoterpene having a hydroxyl group such as citronellol, nerol, geraniol, linalol, lilac alcohol, and nerolidol; a linear monoterpene having an aldehyde group such as citronellal and citral; a linear monoterpene having a ketone group such as perilla ketone, elscholtdiaketone, and tagetone; a linear monoterpene having an oxide group such as rose oxide; a linear monoterpene having a carboxyl group such as a citronellic acid; a hydrocarbon linear monoterpene such as myrcene and ocimene;

a monocyclic monoterpene having a hydroxyl group such as menthol,terpineol, thymol, carvacrol, grandiol, eugenol, and carveol; a monocyclic monoterpene having an aldehyde group such as picrocrocin, perillyl aldehyde, ylide dial, neptalactone, cyclocitral, safranal, phellandral, and naucredal; a monocyclic monoterpene having a ketone group such as menthone, carvomenthone, p-menthane-8-thiol-3-one, nepetalactone, ylide myrmecin, naucredal, carron, irone, ionone, carvotanacetone, and piperitenone; a monocyclic monoterpene having an oxide group such as lineatine, cineol, pinol, and ascaridole; a hydrocarbon monocyclic monoterpene such as menthofuran, terpinene, limonene, terpinolene, phellandrene, and tricyclene;

a bicyclic monoterpene having a hydroxyl group such as borneol, paeoniflorine, pinocarveol, pinocampheol, and twill alcohol; a bicyclic monoterpene having a ketone group such as thujone, umbellone, cull-3-en-2-one, verbenone, and camphor; a bicyclic monoterpene having an oxide group such as chrysanthenol-O-β-D-glucopyranoside; a hydrocarbon bicyclic monoterpene such as thujene, cullen, pinene, and sabinene;

a modified monoterpene having a hydroxyl group such as phentyl alcohol and noj igiku alcohol; a modified monoterpene having a ketone group such as fenchone and santhenone; a modified monoterpene having an ester group such as filifolide; and a hydrocarbon modified monoterpene such as camphene, fentin, and santhen.

Among the above monoterpenes, further preferred is a linear monoterpene having an aldehyde group or a monocyclic monoterpene having a hydroxyl group. As a linear monoterpene having an aldehyde group, citronellal is particularly preferable. As a monocyclic monoterpene having a hydroxyl group, menthol or terpineol is particularly preferable, and menthol is most preferable.

An essential oil containing a terpene can be used instead of a monoterpene. Examples of the essential oil containing a terpene include ylang ylang oil (for example, containing eugenol, linalol), rose oil (for example, containing citronellol, geraniol, nerol), neroli oil (for example, containing linalol), clove oil (for example, containing eugenol), jasmine oil (for example, containing linalol), lemon oil (for example, containing limonene, geraniol, citral, linalol), bergamot oil (for example, containing limonene), lime oil (for example, containing limonene, citral), citronella oil (for example, containing citronellal, geraniol, citronellol), lemon grass oil (for example, containing citral), peppermint oil (for example, containing menthol, menthone), mentha oil (for example, containing menthol), spearmint oil (for example, containing limonene), Japanese cypress oil (for example, containing pinene, limonene, borneol), camphor oil (for example, containing cineol), and Peru balsam (for example, containing nerolidol). Citronella oil, peppermint oil, or mentha oil is particularly preferable.

The content of a monoterpene in the total amount of the external preparation of the present invention is preferably 0.01 to 15% by mass, more preferably 0.01 to 10% by mass, and particularly preferably 0.1 to 10% by mass. When the content of a monoterpene is less than 0.01% by mass, percutaneous absorbability of the compound represented by general formula (1) may not improve, and when the content is more than 15% by mass, skin irritant may occur.

The mass ratio of a compound represented by general formula (1) or a salt thereof to a monoterpene in the external preparation of the present invention is preferably 2000:1 to 1:1500, more preferably 1000:1 to 1:1000, and particularly preferably 50:1 to 1:1000.

It should be noted that formulation of a absorbefacient, for example, a saturated fatty acid such as stearic acid, an unsaturated fatty acid such as oleic acid, an ester such as diisopropyl adipate, a surfactant such as sorbitanmonooleate, a keratin softening agent such as urea, a fatty acid salt such as magnesium stearate, a cyclic polysaccharide such as α-cyclodextrin, an amine such as diphenylamine, an amino acid such as L-arginine acid, and a ketone such as crotamiton in an external preparation, instead of the above monoterpenes, was not able to improve percutaneous absorbability.

A balance amount of water is contained in the external preparation of the present invention. The content in the total amount of the external preparation is preferably 0.01 to 90% by mass, more preferably 1 to 80% by mass, and particularly preferably 10 to 70% by mass.

The pH of the external preparation of the present invention is preferably 6 to 10, more preferably 6 to 9, and particularly preferably 6 to 8.5.

Other optional ingredients that are usually used in a pharmaceutical composition can be added to the external preparation of the present invention in an amount as long as it does not affect the effect of the present invention. Examples of such optional ingredients include solvents, water-soluble polymers, surfactants, stabilizers, pH adjusting agents, crosslinkers, adhesives, tackifiers, plasticizers, and bases.

Examples of the solvents include a monovalent alcohol such as benzyl alcohol, stearyl alcohol, and oleyl alcohol; and a polyvalent alcohol such as concentrated glycerin, polyethylene glycol, 1,3-butylene glycol, 2-ethyl-1,3-hexanediol, and Polypropylene glycol 2000.

Examples of the water-soluble polymers include a cellulose such as hydroxyethylcellulose, hydroxypropylcellulose, and hydroxy propyl methyl cellulose; a polysaccharide such as saccharose; a sugar alcohol such as sorbitol and mannitol; and a synthetic polymer such as polyvinyl alcohol and polyvinylpyrrolidone.

Examples of the surfactants include an anion surfactant such as calcium stearate, magnesium stearate, and sodium laurylsulfate; a cation surfactant such as benzalkonium chloride, benzethonium chloride, and cetylpyridinium chloride; a nonionic surfactant such as glyceryl monostearate, sucrose fatty acid ester, polyoxyethylene hydrogenated castor oil, polyoxyethylene sorbitan fatty acid ester, polyoxyethylene fatty acid ester, and polyoxyethylene alkyl ether.

Examples of the stabilizers include a phenolic compound such as methyl parahydroxybenzoate and propyl parahydroxybenzoate; a neutral compound such as chlorobutanol and phenylethyl alcohol; a cationic soap such as benzalkonium chloride and benzethonium chloride; an antioxidant such as butylhydroxyanisol, tocopherol acetate, propyl gallate, and 2-mercaptobenzimidazole; a reducing agent such as ascorbic acid, sodiumbisulfite, andsodiumthiosulfate; andachelating agent such as lecithin and EDTA.

Examples of the pH adjusting agents include phosphoric acid, boric acid, acetic acid, lactic acid, succinic acid, citric acid, tartaric acid, phthalic acid, their salts such as those with alkalimetals, triethanolamine, diethanolamine, diisopropanolamine, glycine, and sodium hydroxide. Further, Britton-Robinson buffer, Clark-Lubs buffer, Kolthoff buffer, or the like can be used.

Examples of the crosslinkers include magnesium chloride, calcium chloride, aluminium chloride, magnesium oxide, calciumoxide, aluminiumoxide, potassiumalum, driedaluminum hydroxide gel, calcium phosphate, magnesium phosphate, aluminum phosphate, calcium citrate, aluminum acetate, aluminum glycinate, hydrated aluminum silicates, magnesium aluminometasilicate, aluminum lactate, and synthetic hydrotalcite.

Examples of the adhesives include partially neutralized polyacrylic acid, polyacrylic acid or its salts, carboxymethylcellulose or its salts, styrene-isoprene-styrene block copolymers, polyisobutylene, isoprene rubber, styrene-butadiene-styrene copolymers, acrylicpolymers (copolymers consisting of two or more selected from 2-ethylhexyl acrylate, vinyl acetate, ethyl acrylate, ethyl methacrylate, methoxyethyl acrylate, and acrylic acid), and dimethylpolysiloxane.

Examples of the tackifiers include polyterpene resin, petroleumresin, rosin, rosinester, oil soluble phenolic resin tackifiers.

Examples of the plasticizers include liquid paraffin and squalane.

Examples of the bases include sodium alginate, polyethylene oxide, carboxymethyl cellulose, xanthan gum, gum arabic, gum tragacanth, carboxyvinyl polymers, gelatin, starch, kaolin, and titanium oxide.

The form of the present invention is not limited as long as it is an external preparation. The examples include liquid preparations, gels, creams, lotions, sprays, ointments, poultices, and plasters.

Examples

The present invention will be described in detail below by way of Examples. However, it is not intended to limit the present invention to these Examples.

Example 1 [Liquid preparation]

(1) Pitavastatin calcium (0.5 parts by mass) was dissolved in 5 parts by mass of Polyethyleneglycol 400, and then 30 parts by mass of ethanol and 2 parts by mass of 1-menthol (1-menthol; from TAKASAGO INTERNATIONAL CORPORATION) were added before stirring.

(2) Hot purified water (16 parts by mass) was added to 1.3 parts by mass of Hydroxypropylmethylcellulose 2906 before cooling. The mixture was added to (1), and then Britton-Robinsonbuffer (pH 7) was added until the total

amount reached 100 parts by mass to obtain a liquid preparation of the present invention.

Example 2 [Liquid preparation]

A liquidpreparation of the present invention was obtained in the same manner as in Example 1 except that the formulated amount of 1-menthol was changed to 5 parts by mass.

Example 3 [liquid preparation]

(1) Pitavastatin calcium (0.5 parts by mass) was dissolved in 5 parts by mass of Polyethyleneglycol 400, and then 30 parts by mass of ethanol and 2 parts by mass of terpineol (terpineol; from Wako Pure Chemical Industries, Ltd.) were added before stirring.
(2) Hot purified water (16 parts by mass) was added to 1.3 parts by mass of Hydroxypropylmethylcellulose 2906 before cooling. The mixture was added to (1), and then 0.01 parts by mass of phosphoric acid and Britton-Robinson buffer (pH 7) were added until the total amount reached 100 parts by mass to obtain a liquid preparation of the present invention.

Example 4 [Liquid preparation]

A liquid preparation of the present invention was obtained in the same manner as in Example 3 except that terpineol was changed to citronellal (3,7-dimethyl-6-octenal; from Wako Pure Chemical Industries, Ltd.).

Example 5 [Liquid preparation]

(1) Atorvastatin calcium (0.1 parts by mass) was dissolved in 5 parts by mass of Polyethyleneglycol 400, and then 30 parts by mass of ethanol and 2 parts by mass of 1-menthol were added before stirring.
(2) Hot purified water (16 parts by mass) was added to 1.3 parts by mass of Hydroxypropylmethylcellulose 2906 before cooling. The mixture was added to (1), and then 0.03 parts by mass of phosphoric acid and Britton-Robinson buffer (pH 7) were added until the total amount reached 100 parts by mass to obtain a liquid preparation of the present invention.

Example 6 [Liquid preparation]

A liquidpreparation of the present invention was obtained in the same manner as in Example 1 except that pitavastatin calcium was changed to pravastatin sodium.

Comparative Example 1 [Liquid preparation]

A liquid preparation was obtained in the same manner as in Example 1 except that menthol was not formulated therein.

Comparative Example 2 [Liquid preparation]

(1) Pitavastatin calcium (0.5 parts by mass) was dissolved in 5 parts by mass of Polyethyleneglycol 400, and then 30 parts by mass of ethanol and 2 parts by mass of stearic acid (Stearic acidNAA-175; from NOF CORPORATION) were addedbefore stirring.
(2) Hot purified water (16 parts by mass) was added to 1.3 parts by mass of Hydroxypropylmethylcellulose 2906 before cooling. The mixture was added to (1), and then 0.39 parts by mass of a 2 mol/L aqueous potassium hydroxide solution and Britton-Robinson buffer (pH 7) were added until the total amount reached 100 parts by mass to obtain a liquidpreparation. Comparative Examples 3 to 6, 8, 9, 11 [Liquid preparation]

Each liquid preparation was obtained in the same manner as in Comparative Example 2 except that stearic

acidwas changed to oleic acid (oleic acid; from NOF CORPORATION) [Comparative Example 3], diisopropyl adipate (diisopropyl adipate; from Nihon Surfactant Kogyo K.K.) [Comparative Example 4], sorbitan monooleate (Nikkol SO-10; from Nihon Surfactant Kogyo K.K.) [Comparative Example 5], urea (urea; from Takasugi Pharmaceutical Co., Ltd.) [Comparative Example 6], α-cyclodextrin (Celdex; from NIHON SHOKUHIN KAKO CO., LTD.) [Comparative Example 8], diphenylamine (diphenylamine; from KANTO KAGAKU) [Comparative Example 9], and crotamiton (crotamiton; from KONGO CHEMICAL CO., LTD) [Comparative Example 11].

Comparative Example 7 [Liquid preparation]

(1) Pitavastatin calcium (0.5 parts by mass) was dissolved in 5 parts by mass of Polyethyleneglycol 400, and then 30 parts by mass of ethanol and 2 parts by mass of magnesium stearate (magnesium stearate; from NOF CORPORATION) were added before stirring.
(2) Hot purified water (16 parts by mass) was added to 1.3 parts by mass of Hydroxypropylmethylcellulose 2906 before cooling. The mixture was added to (1), and then 0.06 parts by mass of phosphoric acid, 0.03 parts by mass of a 2 mol/L aqueous potassium hydroxide solution, and Britton-Robinson buffer (pH 7) were added until the total amount reached 100 parts by mass to obtain a liquid preparation.

Comparative Example 10 [Liquid preparation]

A liquid preparation was obtained in the same manner as in Example 3 except that terpineol was changed to L-arginine (L-arginine; from KANTO KAGAKU), and the added amount of phosphoric acid was changed to 1.26 parts by mass. Comparative Example 12 [Liquid preparation]
A liquid preparation was obtained in the same manner as in Example 5 except that 1-menthol was not formulated therein.

Comparative Example 13 [Liquid preparation]

A liquid preparation was obtained in the same manner as in Comparative Example 1 except that pitavastatin calcium was changed to pravastatin sodium.

Test Example 1

Skin permeability of the prepared liquid preparations was measured by means of the following method.

(1) Water (9 g) was added to 1 g each of the respective liquid preparations obtained in the above Examples 1 to 6 and Comparative Examples 1 to 13 before shaking. The pH of the respective solutions was then measured at 25°C by a pH meter (HORIBA: F-24).
(2) The respective prepared liquid preparations were used as a donor solution, and Britton-Robinson buffer (pH7) was used as a receptor solution. Excised abdominal skin from a Wistar rat (male, 8 weeks old) was used as a permeable membrane. The skin was placed on a permeable portion of a vertical diffusion cell (Franz cell) with the surface to the donor side, and 1 mL of the donor solution and 30 mL of the receptor solution were filled in the cell. The vertical diffusion cell was maintained at a constant temperature (32°C), and a permeation experiment was performed. In order to prevent moisture evaporation, a donor cell and a sampling portion were covered with a film (PARAFILM; from American National Can Company). The receptor solution (0. 5 mL) was collected from the sampling portion every two hours, and 0.5 mL of a new receptor solution was added.

Tert-butylmethylether was added to the collected receptor solution, and pitavastatin or atorvastatin was extracted. Then, pitavastatin or atorvastatin was quantified by an HPLC method (internal standard substance: (E)-3(R),5 (S)-dihydroxy-7-(2'-isopropyl-4'-(4"-fluorophen yl)quinolin-3'-yl)-6-heptenoic acid, measured wavelength: 245 nm, column: Develosil ODS-HG-5, column temperature: 40°C, mobile phase: 0.2 mol/L acetic acid/acetonitrile/methanol = 60/30/10).
An acetonitrile/water (3/2) solution was added to the collected receptor solution. Then, pravastatin was quantified by an HPLC method (internal standard: ethylparaben, measured wavelength: 238 nm, column: Develosil ODS-HG-5, temperature: 40°C, mobile phase: water/methanol/acetic acid/triethylamine = 550/450/1/1).
Permeability coefficient was calculated from the concentration of pitavastatin, atorvastatin, or pravastatin in accordance with the following equation, and skin permeability was evaluated. The results are shown in Tables 1 to 3.

(Mathematical expression)

Permeability coefficient (cm/h) = Flux at a steady state ($g/m^2 \cdot h$)/donor drug concentration ($\mu g/m^3$)

Table 1

| (parts by mass) | | | | | | |
|---|---|---|---|---|---|---|
| Ingredient | Example | | | | | |
| | 1 | 2 | 3 | 4 | 5 | 6 |
| Pitavastatin calcium | 0.5 | 0.5 | 0.5 | 0.5 | - | - |
| Atorvastatin calcium | - | - | - | - | 0.1 | - |
| Pravastatin sodium | - | - | - | - | - | 0.5 |
| Hydroxypropylmethylcellulose 2906 | 1.3 | 1.3 | 1.3 | 1.3 | 1.3 | 1.3 |
| Polyethyleneglycol 400 | 5 | 5 | 5 | 5 | 5 | 5 |
| Ethanol | 30 | 30 | 30 | 30 | 30 | 30 |
| Purified water | 16 | 16 | 16 | 16 | 16 | 16 |
| Phosphoric acid | - | - | 0.01 | 0.01 | 0.03 | - |
| 2 mol/L aqueous potassium hydroxide solution | - | - | - | - | - | - |
| 1-menthol | 2 | 5 | - | - | 2 | 2 |
| Terpineol | - | - | 2 | - | - | - |
| Citronellal | - | - | - | 2 | - | - |
| Stearic acid | - | - | - | - | - | - |
| Oleic acid | - | - | - | - | - | - |
| Diisopropyl adipate | - | - | - | - | - | - |
| Sorbitan monooleate | - | - | - | - | - | - |
| Urea | - | - | - | - | - | - |
| Magnesium stearate | - | - | - | - | - | - |
| $\alpha$-cyclodextrin | - | - | - | - | - | - |
| Diphenylamine | - | - | - | - | - | - |
| L-arginine | - | - | - | - | - | - |
| Crotamiton | - | - | - | - | - | - |
| Britton-Robinson buffer (pH 7) | Total amount 100 | Total amount 100 | Total amount 100 | Total amount 100 | Total amount 100 | Total amount 100 |
| pH (immediately after preparation) | 7.1 | 7.0 | 6.9 | 7.0 | 6.9 | 7.1 |

(continued)

| Ingredient | Example | | | | | |
|---|---|---|---|---|---|---|
| | 1 | 2 | 3 | 4 | 5 | 6 |
| Permeability coefficient ($10^{-4}$ cm/h) | 12.9 | 31.4 | 25.4 | 19.3 | 35.8 | 12.9 |

Table 2

(parts by mass)

| Ingredient | Comparative Example | | | | | | |
|---|---|---|---|---|---|---|---|
| | 1 | 2 | 3 | 4 | 5 | 6 | 7 |
| Pitavastatin calcium | 0.5 | 0.5 | 0.5 | 0.5 | 0.5 | 0.5 | 0.5 |
| Atorvastatin calcium | - | - | - | - | - | - | - |
| Pravastatin sodium | - | - | - | - | - | - | - |
| Hydroxypropylmethyl cellulose 2906 | 1.3 | 1.3 | 1.3 | 1.3 | 1.3 | 1.3 | 1.3 |
| Polyethyleneglycol 400 | 5 | 5 | 5 | 5 | 5 | 5 | 5 |
| Ethanol | 30 | 30 | 30 | 30 | 30 | 30 | 30 |
| Purified water | 16 | 16 | 16 | 16 | 16 | 16 | 16 |
| Phosphoric acid | - | - | - | - | - | - | 0.06 |
| 2 mol/L aqueous potassium hydroxide solution | - | 0.39 | 1.29 | 0.03 | 0.03 | 0.03 | 0.03 |
| 1-menthol | - | - | - | - | - | - | - |
| Terpineol | - | - | - | - | - | - | - |
| Citronellal | - | - | - | - | - | - | - |
| Stearic acid | - | 2 | - | - | - | - | - |
| Oleic acid | - | - | 2 | - | - | - | - |
| Diisopropyl adipate | - | - | - | 2 | - | - | - |
| Sorbitan monooleate | - | - | - | - | 2 | - | - |
| Urea | - | - | - | - | - | 2 | - |
| Magnesium stearate | - | - | - | - | - | - | 2 |
| α-cyclodextrin | - | - | - | - | - | - | - |
| Diphenylamine | - | - | - | - | - | - | - |
| L-arginine | - | - | - | - | - | - | - |
| Crotamiton | - | - | - | - | - | - | - |
| Britton-Robinson buffer (pH 7) | Total amount 100 | Total amount 100 | Total amount 100 | Total amount 100 | Total amount 100 | Total amount 100 | Total amount 100 |
| pH (immediately after preparation) | 6.9 | 7.1 | 7.0 | 6.9 | 6.9 | 7.0 | 7.0 |

(continued)

| Ingredient | Comparative Example | | | | | | |
|---|---|---|---|---|---|---|---|
| | 1 | 2 | 3 | 4 | 5 | 6 | 7 |
| Permeability coefficient ($10^{-4}$ cm/h) | 0.8 | 1.5 | 6.1 | 3.7 | 0.7 | 1.0 | 0.8 |

Table 3

(parts by mass)

| Ingredient | Comparative Example | | | | | |
|---|---|---|---|---|---|---|
| | 8 | 9 | 10 | 11 | 12 | 13 |
| Pitavastatin calcium | 0.5 | 0.5 | 0.5 | 0.5 | - | - |
| Atorvastatin calcium | - | - | - | - | 0.1 | - |
| Pravastatin sodium | - | - | - | - | - | 0.5 |
| Hydroxypropylmethylcellulose 2906 | 1.3 | 1.3 | 1.3 | 1.3 | 1.3 | 1.3 |
| Polyethyleneglycol 400 | 5 | 5 | 5 | 5 | 5 | 5 |
| Ethanol | 30 | 30 | 30 | 30 | 30 | 30 |
| Purified water | 16 | 16 | 16 | 16 | 16 | 16 |
| Phosphoric acid | - | - | 1.26 | - | 0.03 | - |
| 2 mol/L aqueous potassium hydroxide solution | 0.03 | 0.06 | - | 0.03 | - | - |
| 1-menthol | - | - | - | - | - | - |
| Terpineol | - | - | - | - | - | - |
| Citronellal | - | - | - | - | - | - |
| Stearic acid | - | - | - | - | - | - |
| Oleic acid | - | - | - | - | - | - |
| Diisopropyl adipate | - | - | - | - | - | - |
| Sorbitan monooleate | - | - | - | - | - | - |
| Urea | - | - | - | - | - | - |
| Magnesium stearate | - | - | - | - | - | - |
| $\alpha$-cyclodextrin | 2 | - | - | - | - | - |
| Diphenylamine | - | 2 | - | - | - | - |
| L-arginine | - | - | 2 | - | - | - |
| Crotamiton | - | - | - | 2 | - | - |
| Britton-Robinson buffer (pH 7) | Total amount 100 | Total amount 100 | Total amount 100 | Total amount 100 | Total amount 100 | Total amount. 100 |
| pH (immediately after preparation) | 7.0 | 7.1 | 7.1 | 7.0 | 7.0 | 6.9 |
| Permeability coefficient ($10^{-4}$ cm/h) | 0.7 | 1.0 | 0.9 | 1.2 | 0.9 | 0.2 |

In the liquid preparations of the present invention in which 1-menthol (Examples 1 and 2), terpineol (Example 3), and citronellal (Example 4) were formulated, skinpermeability of pitavastatin calcium was high. However, in the liquid preparation in which 1-menthol was not formulated (Comparative Example 1) and the liquid preparations in which stearic acid (Comparative Example 2), oleic acid (Comparative Example 3), diisopropyl adipate (Comparative Example 4), sorbitan monooleate (Comparative Example 5), urea (Comparative Example 6), magnesium stearate (Comparative Example 7), α-cyclodextrin (Comparative Example 8), diphenylamine (Comparative Example 9), L-arginine (Comparative Example 10), and crotamiton (Comparative Example 11) were formulated instead of 1-menthol, each skin permeability of pitavastatin calcium was low.

In the liquid preparations of the present invention (Examples 5 and 6) in which 1-menthol was formulated, skin permeability of atorvastatin calcium or pravastatin sodium was high. However, in the liquid preparations in which 1-menthol (Comparative Examples 12 and 13) was not formulated, the skin permeability was low.

Example 7 [Poultice]

(1) Pitavastatin calcium (0.5 parts by mass) and 2 parts by mass of 1-menthol were dissolved in 10 parts by mass of Polyethyleneglycol 400, and then 15 parts by mass of concentrated glycerin, 0.1 parts by mass of Polysolvate 80, 4 parts by mass of carmellose sodium, 0.75 parts by mass of magnesium aluminometasilicate, 2 parts by mass of sodium polyacrylate, and 2 parts by mass of partially neutralized polyacryl ic acid were addedbefore uniformly stirring to obtain an oil phase.

(2) Sodium edetate (0.1 parts by mass) and 0.25 parts by mass of tartaric acid were dissolved in 35 parts by mass of purified water, and then 3 parts by mass of kaolin and 25 parts by mass of a D-sorbitol solution (70%) were added before uniformly stirring to obtain an aqueous phase.

(3) The aqueous phase was added to the oil phase, and purified water was added until the total amount reached 100 parts by mass before mixing for 10 minutes (revolution: 40 rpm, rotation: 80 rpm) using a mixing machine (from KODAIRA SEISAKUSHO, LTD.) to produce a patch material.

(4) The patch material was spread between a nonwoven fabric and a liner to a thickness of 1 mm using a spread machine (from IPROS CO., Ltd.) to produce a poultice of the present invention.

Example 8 [Poultice]

A poultice of the present invention was produced in the same manner as in Example 7 except that pitavastatin calcium was changed to atorvastatin calcium, and the formulated amount was changed to 0.1 parts by mass.

Comparative Example 14 [Poultice]

A poultice was produced in the same manner as in Example 7 except that 1-menthol was not formulated.

Comparative Example 15 [Poultice]

A poultice was produced in the same manner as in Example 8 except that 1-menthol was not formulated.

Text Example 2

(1) The poultices produced in Examples 7 and 8, and Comparative Examples 14 and 15 (3.5 cm x 2.0 cm) were cut into small strips using a pair of scissors, and then 20 mL of purified water was added before shaking for 30 minutes. The pH was measured at 25°C by a pH meter (HORIBA: F-24).

(2) Pitavastatin calcium or atorvastatin calcium was quantified in the same manner as for the aforementioned skin permeability except that the poultices produced in Examples 7 and 8, and Comparative Examples 14 and 15 were applied to the donor side to calculate permeability coefficients. The results are shown in Table 4.

Table 4

| |
|---|
| (parts by mass) |

(continued)

| Ingredient | Example | | Comparative Example | |
|---|---|---|---|---|
| | 7 | 8 | 14 | 15 |
| Pitavastatin calcium | 0.5 | - | 0.5 | - |
| Atorvastatin calcium | - | 0.1 | - | 0.1 |
| 1-menthol | 2 | 2 | - | - |
| Polyethyleneglycol 400 | 10 | 10 | 10 | 10 |
| Concentrated glycerin | 15 | 15 | 15 | 15 |
| D-sorbitol solution (70%) | 25 | 25 | 25 | 25 |
| Kaolin | 3 | 3 | 3 | 3 |
| Carmellose sodium | 4 | 4 | 4 | 4 |
| Magnesium aluminometasilicate | 0.75 | 0.75 | 0.75 | 0.75 |
| Sodium edetate | 0.1 | 0.1 | 0.1 | 0.1 |
| Sodium polyacrylate | 2 | 2 | 2 | 2 |
| Partially neutralized polyacrylic acid | 2 | 2 | 2 | 2 |
| Tartaric acid | 0.25 | 0.25 | 0.25 | 0.25 |
| Polysolvate 80 | 0.1 | 0.1 | 0.1 | 0.1 |
| Purified water | Total amount 100 | Total amount 100 | Total amount 100 | Total amount 100 |
| pH (immediately after preparation) | 7.2 | 6.9 | 7.2 | 7.0 |
| Permeability coefficient ($10^{-4}$ cm/h) | 1.3 | 2.7 | 0.2 | 0.0 |

In the poultice in which 1-menthol was formulated (Example 7), skin permeability of pitavastatin calcium was high, but in the poultice in which 1-menthol was not formulated (Comparative Example 14), skin permeability of pitavastatin calcium was low. Therefore it was proved also in a poultice that formulation of 1-menthol improved the skin permeability. The effect of 1-menthol was proved also for atorvastatin calcium in the same manner.

Example 9 [Gel]

Pitavastatin calcium (0.5 parts by mass) and 2 parts by mass of mentha oil were dissolved in 10 parts by mass of Polyethyleneglycol 400 to obtain an oil phase. Separately, 0.5 parts by mass of a carboxyvinyl polymer (Ultrez 10: BF; from Goodrich Corporation) was allowed to swell in 40 parts by mass of Britton-Robinson buffer (pH7) to obtain an aqueous phase. The aqueous phase was added to the oil phase, and the buffer was added until the total amount reached 100 parts by mass to produce a gel of the present invention.

Example 10 [Cream]

Pitavastatin calcium (0.5 parts by mass), 0.5 parts by mass of polyoxyethylene sorbitan monooleate (20 EO), and 2 parts by mass of peppermint oil were dissolved in 10 parts by mass of Polyethyleneglycol 400 to obtain an oil phase. Separately, 0. 5 parts by mass of a carboxyvinyl polymer (Ultrez 10: BF; from Goodrich Corporation) was allowed to swell in 25 parts by mass of Britton-Robinsonbuffer to obtain an aqueous phase. The aqueous phase was added to the oil phase, and the buffer was added until the total amount reached 100 parts by mass to produce a cream of the present invention.

Example 11 [Plaster]

(1) Liquid paraffin (37.5 parts by mass) was added to 20 parts by mass of a styrene-isoprene-styrene copolymer (Quintac 3570C; from ZEON CORPORATION) and 35 parts by mass of alicyclic saturated hydrocarbon resin (Alcon P-100; from Arakawa Chemical Industries, Ltd.). The mixture was heated to 150°C and dissolved.
(2) Pitavastatin calcium (0.5 parts by mass), 5 parts by mass of polyoxyethylenelaurylether (Nikkol BL-9EX; from Nihon Surfactant Kogyo K.K.), and 2 parts by mass of 1-menthol were heated to 40°C and dissolved.
(3) (2) was added to (1), and the mixture was stirred by a glass rod to combine the entire mixture uniformly, and spread by a spreading machine and then cooled to produce a plaster of the present invention.

Each dosage form of a gel (Example 9), a cream (Example 10), and a plaster (Example 11) had excellent percutaneous absorbability.

## Claims

1. An external preparation comprising following ingredients (A) and (B) :

   (A) a compound represented by the following general formula (1)

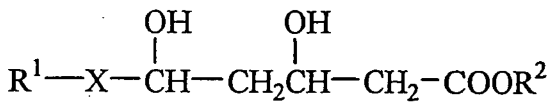

(1)

   wherein $R^1$ represents an organic residue having a cyclic structure that may have a substituent; $R^2$ represents a hydrogen atom or a lower alkyl group; and X represents an ethylene group or an ethenylene group
   or a salt thereof, and
   (B) a monoterpene.

2. The external preparation according to claim 1, wherein ingredient (A) is lovastatin, simvastatin, pravastatin, fluvastatin, atorvastatin, serivastatin, mevastatin, rosuvastatin, or pitavastatin, or their salts.

3. The external preparation according to claim 1 or 2, wherein ingredient (A) ispitavastatin, atorvastatin, orpravastatin, or their salts.

4. The external preparation according to any one of claims 1 to 3, wherein ingredient (A) is pitavastatin calcium, atorvastatin calcium, or pravastatin sodium.

5. The external preparation according to any one of claims 1 to 4, wherein the monoterpene is a monoterpene having an aldehyde group or a monoterpene having a hydroxyl group.

6. The external preparation according to any one of claims 1 to 5, wherein the monoterpene is a linear monoterpene having an aldehyde group or a monocyclic monoterpene having a hydroxyl group.

7. The external preparation according to claim 6, wherein the linear monoterpene having an aldehyde group or the monocyclic monoterpene having a hydroxyl group is one or more materials selected from menthol, terpineol, and citronellal.

8. The external preparation according to any one of claims 1 to 7, comprising 0.001 to 20% by mass of ingredient (A).

9. The external preparation according to any one of claims 1 to 8, comprising 0.01 to 15% by mass of the monoterpene.

**INTERNATIONAL SEARCH REPORT**

International application No.
PCT/JP2005/006038

A. CLASSIFICATION OF SUBJECT MATTER
Int.Cl[7] A61K31/22, 31/40, 31/47, 47/08, 47/10, A61P17/00

According to International Patent Classification (IPC) or to both national classification and IPC

B. FIELDS SEARCHED

Minimum documentation searched (classification system followed by classification symbols)
Int.Cl[7] A61K31/22, 31/40, 31/47, 47/08, 47/10, A61P17/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAPLUS(STN), MEDLINE(STN), BIOSIS(STN), EMBASE(STN)

C. DOCUMENTS CONSIDERED TO BE RELEVANT

| Category* | Citation of document, with indication, where appropriate, of the relevant passages | Relevant to claim No. |
|---|---|---|
| Y | JP 10-505838 A (RAMOT UNIVERSITY AUTHORITY FOR APPLIED RESEARCH AND INDUSTRIAL DEVELOPMEN LTD.), 09 June, 1998 (09.06.98), Full text & WO 96/08248 A1 & EP 793489 A1 | 1-9 |
| Y | JP 2001-507331 A (LTS LOHMANN THERPIE-SYSTEME GMBH), 05 June, 2001 (05.06.01), Full text & WO 97/17061 A1 & EP 859595 A1 & US 6379696 B1 | 1-9 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 April, 2005 (19.04.05) | 10 May, 2005 (10.05.05) |
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2005/006038

C (Continuation). DOCUMENTS CONSIDERED TO BE RELEVANT

| Category* | Citation of document, with indication, where appropriate, of the relevant passages | Relevant to claim No. |
|---|---|---|
| Y | WO 94/16682 A (Yamanouchi Pharmaceutical Co., Ltd.), 04 August, 1994 (04.08.94), Full text & EP 682942 A1 | 1-9 |
| A | JP 9-143062 A (Mikasa Seiyaku Kabushiki Kaisha), 03 June, 1997 (03.06.97), Full text (Family: none) | 1-9 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5856336 A
- US 4346227 A
- US 6080778 A
- US 6472421 B
- US 6511800 B
- US 6022887 A
- US 5730992 A
- US 6126947 A
- US 5840752 A
- US 5733558 A
- US 5902805 A
- US 4739073 A
- EP 114027 A
- EP 367895 A
- US 5001255 A
- US 4613610 A
- US 4851427 A
- US 4755606 A
- US 4808607 A
- US 4751235 A
- US 4939159 A
- US 4822799 A
- US 4804679 A
- US 4876280 A
- US 4829081 A
- US 4927851 A
- US 4588715 A
- EP 304063 A
- EP 330057 A
- US 5026708 A
- US 4868185 A
- EP 324347 A
- EP 300278 A
- US 5013749 A
- US 5872130 A
- US 4231938 A
- US 4444784 A
- US 5354772 A
- US 5273995 A
- US 5177080 A
- US 3983140 A
- JP 2648897 B
- US 5260440 A
- JP 2569746 B

### Non-patent literature cited in the description

- **F. G. KATHAWALA.** *Medical Research Reviews,* 1991, vol. 11, 121-146
- *Bioorganic & Medicinal Chemistry,* 1977, vol. 5, 437